# EUROPEAN PATENT SPECIFICATION

(11) **EP 1 603 615 B1**
(45) Date of publication and mention of the grant of the patent: **09.07.2008**
(21) Application number: 04721094.3
(22) Date of filing: 17.03.2004
(51) Int. Cl.: A61M 15/00

(54) **IMPROVED DRY POWDER INHALER SYSTEM**
VERBESSERTES TROCKENPULVER-INHALATIONSSYSTEM
SYSTEME D'INHALATEUR DE POUDRE SECHE AMELIORE

(30) Priority: 20.03.2003 WO PCT/BE03/00048
(43) Date of publication of application: 14.12.2005
(73) Proprietor: Galephar M/F, 6900 Marche-en-Famenne (BE)
(72) Inventor: VANDERBIST, Francis, B-1650 Beersel (BE); BAUDIER, Philippe, B-1180 Uccle (BE); DEBOECK, Arthur, Gurabo, Puerto Rico00778 (US)
(74) Representative: Vandeberg, Marie-Paule L.G.
(86) International application number: PCT/BE2004/000039
(87) International publication number: WO 2004/082750

(56) References cited:
- EP-A- 0 606 486
- EP-A- 0 666 085
- US-A- 5 715 810

## Description

### ABSTRACT

The present invention relates to a pharmaceutical composition for inhalation, consisting in a combination of (A) a dry powder formulation containing a micronized active ingredient, alone or mixed with an inactive ingredient, said powder being filled in a hydroxypropylmethylcellulose (HPMC) capsule and (B) a single dose dry powder inhaler device especially adapted to said capsule to provide a high respiratory dose of said active ingredient when said drug is inhaled by the mouth through said device. Said device being characterized in that he is equipped with piercing needles or pins (in order to pierce the capsule) of diameter of not less than 0.8 mm preferably not less than 1 mm.

### BACKGROUND

Capsules, and essentially hard gelatin capsules are very widely used in the pharmaceutical industry to allow oral administration of drugs.

Hard gelatin capsules were developed as an edible container to mask the taste and odour of medicines. As a result of the introduction of mass-production techniques and high-speed capsule filling machines, capsules have become one of the most popular dosage forms for pharmaceuticals. Capsules have traditionally been used for powder or granule formulations, but later have been adapted to contain oily liquids, tablets and even powders for inhalation. Capsules enjoy widespread popularity because of their relative ease of manufacture (compared with other dosage forms such as tablets) and flexibility of size to accommodate a range of fill weights. They are readily able to achieve bioequivalence between different strengths of the same formulation.

Hard gelatin capsules do have some drawbacks. In capsule shells made from gelatin, the main material used for this purpose generally contains 13-15% water and therefore may not be suitable for use with water sensitive drugs or drug composition. Some drugs may react with the amino groups of gelatin, causing discolouration or formation of crosslinks between gelatin molecules which retard capsule dissolution. Gelatin products are sometimes shunned as a result of religious or vegetarian dietary restrictions.

Hard gelatin capsules capsules have also been used since about 25 years to administer powder for inhalation. In this case, the capsule is pierced using an adequate inhalation device and the powder is inhaled via the mouth or sometimes via the nose.

As said, the main disadvantages of hard gelatin capsules are their relatively high water content (13-16%), their animal origin, their brittleness characteristics and the fact that they may chemically or physically interact with some active or inactive ingredients.

Therefore, more recently, other kinds of pharmaceutical hard capsules have been developed as an alternative to hard gelatin capsules. Among those new hard capsule types, hydroxypropylmethylcellulose (HPMC) also called hypromellose capsules appear to be the most promising. For the clarity of the present text, "HPMC capsules" will be defined as pharmaceutically acceptable capsules containing at least 70 % by weight of hydroxyppropylmethylcellulose.

An example of HPMC capsules useful for pharmaceutical applications are described in US patent 5,756,123. Those HPMC capsules are of vegetal origin and contain 79.6-98.7% by weight of hydroxypropylmethylcellulose, 0.03-0.5% by weight of carrageenan and 0.14-3.19% by weight of a potassium and/or calcium ion.

US 5,715,810 describes a device for oral or nasal inhalation of finely divided materials such as medicinal agents and drugs placed in a hermetically sealed container.
No reference at all is made in said document to the use of HPMC capsules, nor to the advantages to use such capsules, as among others, for the man skilled in the art "hermetically sealed container" does not encompass HPMC capsules.

EP606486 describes a pharmaceutical preparation for inhalation comprising a powdered preparation for intra-tracheal administration contained in a container made for a material mainly comprising at least one compound of the group consisting of hydroxypropylmethylcellulose, methylcellulose, hydroxypropylcellulose, starch, hydroxypropylated starch and sodium alginate. According to said document, less adhesion or absorption of the drug will be achieved in said container, with respect to receptacles made of gelatin, polypropylene, aluminum foil or glass.
Tests made by applicant have shown that when using conventional dry inhalation system with conventional piercing system, after inhalation, the amount of drug still present in the HPMC capsule (about 20% of the drug initially present in the capsule) was substantially equal to the amount of drug still present in the gelatine capsule (about 18% of the drug initially present in the capsule). Tests made by Applicant have also shown that when using a conventional inhaler system, the lung deposition was better when using a gelatin capsule, instead of a HPMC capsule.

It has now been found that by using a HPMC container or capsule with a specific dry powder inhaler, it was possible to increase the lung deposition of the drug, said increased lung deposition being greater than the lung deposition obtained when using a gelatin capsule with a conventional dry powder inhaler, as well as with the specific dry powder inhaler of the invention.
The system of the invention is derived from a device disclosed in US, 3,991,761, which forms the closest prior art for the invention.

### PHYSICAL CHARACTERISTICS OF HPMC CAPSULES

HPMC capsules are odourless and flexible, and exhibit similar dissolution behaviour to the gelatin capsule. Their appearance is similar, except that it lacks the lustre of gelatin. The physical properties of both HPMC and gelatin capsule shells that may affect stability and dissolution, and therefore their suitability for use with various formulations and intended use, are listed in Table I.

**Table 1: Comparative characteristics of HPMC capsules and hard gelatin capsules**

| | **HPMC capsules** | **Gelatin capsules** |
|---|---|---|
| Moisture content | 2-5% | 13-15% |
| Water vapour permeability | Low | Low |
| Substrate for protease | No | Yes |
| Maillard reaction with drug fill | No | Yes |
| Deformation by heat | Above ~ 80°C | Above ~ 60°C (degradation) |
| Water dissolution at room temperature | Soluble | Soluble |
| Static | Low | High |
| Light degradation | No | Possible |

There are two components of capsule shell hardness - brittleness and tolerance to deformation - which determine suitability for use with automated encapsulating machines, as well as end use. When the moisture content of the capsule shell is decreased, as may occur when a desiccant is added to a package of capsules containing moisture-labile drugs, gelatin capsules tend to become brittle and are subject to breakage during transport and storage. The relationship between brittleness and moisture content can be determined using a hardness tester. The results of the testing show that the percentage of broken gelatin capsules sharply increases as the moisture content of the hard gelatin shell drops below 10%, although the degree of brittleness can be modified somewhat by addition of polyethylene glycol (PEG) during manufacture. In contrast, this problem is not observed in HPMC capsule shells even at moisture levels close to 0 %.

Dry Powder Inhalers (DPIs) formulations are more and more used in therapeutics since the Metered Dose Inhalers (MDIs) containing chlorofluorocarbon (CFCs) gases have been shown to provoke a greenhouse effect by destroying the ozone layer.

DPI devices may be either single dose or multidose. In the single dose DPI formulations, the drug is pre-packaged in capsules or blisters. The multidose DPI formulations involve a device containing at least a reservoir and a metering chamber to administer an accurate dose of the drug. A large number of patent applications are submitted each year about new monodose or multidose DPI devices. Monodose and multidose devices each present their own advantages and the final choice for administering the drug via one or the other kind of device is influenced by a large number of parameters as listed hereinbelow :

**Table 2 : Characteristics of the ideal DPI device**

| | |
|---|---|
| 1. | High lung deposition of the drug (with low deposition in the oropharynx and low losses in the device itself) |
| 2. | lung deposition as lowly as possible dependent to the airflow |
| 3. | high inter-doses, inter-patients and inter-devices reproducibility |
| 4. | chemical, physical and microbiological stability of the drug |
| 5. | simplicity of use |
| 6. | portability |
| 7. | acceptability by the patients |
| 8. | low cost of manufacturing |
| 9. | inhalation chamber transparent in order that the patient can check if he has inhaled the whole drug dose (compliance) |

The present invention only relates to monodose DPI devices working with capsules. The single dose DPI devices working with capsules, usually possess a system to pierce the capsule. After the piercing, the patient inhales the powder contained in the capsule through the device without swallowing the capsule. The capsule remains in the device (and is consequently not swallowed by the patient).

It is of the uttermost importance that as much as possible of the medication contained in the capsules is inhaled. Ideally, the capsule should be entirely empty after inhalation. It is the object of the present invention to improve the emptying of the HPMC capsules by the use of a device equipped with piercing pins having a diameter of at least 0.8 mm, said pins being preferably bevel-edged.

### Brief description of the invention

The present invention relates to an improved inhalation pharmaceutical composition consisting of (A) a dry powder formulation containing a micronized active ingredient, alone or mixed with an inactive ingredient, said powder being filled in a hydroxypropylmethylcellulose (HPMC) container or capsule, said container having an outer surface extending between two end portions intended to be pierced or perfored, and (B) a dry powder inhaler device, advantageously a single dose dry powder inhaler device adapted to said capsule and equipped with at least one piercing pin having an equivalent diameter of not less than 0.8 mm.
Advantageously, the piercing pin(s) has an equivalent diameter lower than 3mm, preferably lower than 2mm.
The equivalent diameter of the pin is determined at the section of the pin causing the larger hole in the envelope. The equivalent diameter of said section corresponds to 4 times the cross section area of said section divided by the outer perimeter of said cross section area, wherein the cross section area is determined in a plane perpendicular to the direction of movement of the pin. In case the pin is a rod provided with a shaped cutting or piercing end, the equivalent diameter corresponds to the diameter of the rod.
The cross section area of said section is advantageously circular or substantially circular, but can also be square, rectangular, elliptic, hexagonal, octahedral, pentagonal, etc.
Advantageously, the cross section area of said section is of at least 1.5mm², preferably at least about 1.9mm², such as about 2mm², 2.5mm², 3mm², etc.

According to a preferred embodiment, the piercing systems are bevel-edged needles or pins. This enables an easier piercing of the envelope, even if the diameter of the piercing pin is larger.

The dry powder inhaler is advantageously equipped with at least two advantageously substantially identical piercing systems, adapted to pierce or perfore said container at said two end portions, said piercing systems having an equivalent diameter of not less than 0.8 millimeter (mm), preferably not less than 1 mm, whereby the piercing systems are adapted so that the equivalent diameter of the hole or holes pierced by each piercing system (after removal of the piercing system from the holes) is from 10 to 31 %, and preferably from 15 % to 26 % of the equivalent diameter (advantageously the equivalent inner diameter) of the cross section of the portion of the outer surface of the container (of the hollow or room defined by said outer surface) to be pierced located between the two pierced end portions, said cross section being located in a plane perpendicular to an axis extending between the end portions, advantageously a symmetrical axis extending between the end portions.
Preferably, the improved dry powder inhalation system of the invention comprises :
- at least one micronized active ingredient, optionally in association with excipients, contained in an elongated container which is an hydroxypropylmethylcellulose container, advantageously an elongated capsule, said container having an elongated outer surface with a longitudinal axis of symmetry, said outer surface extending between two curved end portions intended to be pierced or perfored, and
- an dry powder inhaler device equipped with at least two advantageously substantially identical piercing systems, able to pierce or perfore said container at said two end portions, said piercing systems having an equivalent diameter of not less than 0.8 millimeter (mm), preferably not less than 1 mm, whereby the piercing system are adapted so that the equivalent diameter of the holes pierced by each piercing system (after removal of the piercing system of the hole) is from 9 to 30 %, and preferably from 14 % to 25 % of the average equivalent diameter (advantageously the equivalent inner diameter) of the cross section of the portion of the outer surface of the container perpendicular to the longitudinal axis of symmetry .

Advantageously, the number of said piercing systems per device is less than 8, preferably less than 5, more preferably less than 3. Most preferably, the number of piercing systems is 1 or 2, 2 being the most preferred number.

The active ingredient is advantageously mixed with pharmaceutical acceptable carrier before being filled in said container. Said pharmaceutical acceptable carrier is advantageously a mono- or dissacharide derivative and/or a lactose.

The weight mean size of the micronized active(s) ingredient(s) is advantageously below 10 µm, preferably below 8 µm and most preferably below 6 µm. Most preferably, the weight average particle size of the active ingredient is comprised between 0.5 µm and 5µm. According to an embodiment, the capsule contains substantially no particles with a particle size of less than 0.4µm.

According to an embodiment, the micronized active ingredient is from the class of mucolytics, bronchodilators, corticosteroids, xanthine derivatives, leukotriene antagonists, proteins or peptides, and mixtures thereof.

According to a specific embodiment, the active ingredient is L-lysine N-acetylcysteinate.
It is obvious that the dry powder composition can contain two or more active ingredients.

Herein is also disclosed the use of a hydroxypropylmethylcellulose capsule for the preparation of a dosage form containing a dry powder of at least one therapeutic active agent to be administered by inhalation after having pierced holes, each hole having an open passage of at least 1.5 mm², preferably at least 2mm², most preferably at least 2.5 mm² in said hydroxypropylmethylcellulose capsule.

According to a specific embodiment, the dry powder inhalation system comprises at least one buccal or nasal piece and one basal piece adapted for containing the capsule , said basal piece being equipped with two pins and at least a means for actuating or moving said pins towards the envelope to be pierced. The means for moving the two pins is advantageously pressing buttons operating the pins.

The system of the invention may be used for :
- treating respiratory diseases or preventing respiratory troubles ;
- administering one or more active ingredients in the lungs ;
- administering one or more active ingredients in the systemic circulation.

Said procedures comprise advantageously at least the following successive steps : - breaking at least partly an HPMC capsule containing an effective amount of at least a therapeutic active agent, and - administering by inhalation an effective amount of said therapeutic active agent(s).

### Brief description of the drawings

Figure 1 is a schematic view (with cross section) of a four pin device,
Figure 2 is a schematic view with cross section of a "single pin device" of the invention,
Figures 3 and 4 are longitudinal side views of a pin of the device of figure 2,
Figure 5 is an upper view of the basal element of the device of figure 2,
Figure 6 is a cross section view of the basal element of the figure 5 along the line VI-VI,
Figure 7 is a view similar to the figure 6 showing the movement of the two pins,
Figure 8 is a schematic view of a pierced envelope,
Figure 9 is a cross section view of the pierced envelope of figure 8, and
Figure 10 shows a comparison of various FPD, namely the FPD of budesonide/ salmeterol 200 / 25 µg DPI combinations + single pin device versus the FPD obtained Serevent^{®} Diskus^{®} 50 µg and Pulmicort^{®} Turbohaler 200 µg (MLI).

### DETAILED DESCRIPTION OF THE INVENTION

It is known that to reach the lungs, only particles inhaled have a diameter less than 6.0 µm and for reaching the alveoli the diameter should be less than 2.0 µm. Very small particles, having a diameter less than 0.5 µm, are re-exhaled after inhalation. Consequently, the ideal size range for inhaled particles is between 0.5 and 6.0 µm. According to an embodiment, the average particle size (average in weight) is less than 10µm, advantageously less than 8µm, preferably less than 6 µm. According to a specific embodiment, the weight average particle size is comprised between 0.5 µm and 6 µm, preferably lower than 5 µm, most preferably lower than 4µm, such as about 2µm, about 3µm. Advantageously, the average (average in weight) particle size of the active agent containing powder is about 3µm, with at least 50% by weight of the particles have a size comprised between about 2 µm and about 4 µm.

The importance of obtaining a high and reproducible dose of the active substance in the lungs are multiple. First of all, if the lung deposition is high, it may allow to reduce the nominal dose of the drug substance and decreasing the amount of drug absorbed in the systemic circulation and also potentially decrease the side-effects of the drug. This is especially true for corticosteroids and long acting β-2 mimetics which present potentially serious side-effects. On the other hand, the fact to dispose of a DPI system reliable i.e. able to deliver a reproducible dose to the lung is of primary importance for the efficacy of the product in the long term and for the patients to feel reassured about their treatment.

DPI formulations may contain only the micronized active ingredient or the micronized active ingredient mixed with one or more inactive ingredients. The main role of the inactive ingredient is to improve the flowability and the delivery of the dry powder.

The idea of using the HPMC capsules for inhalation is not novel since it has been mentioned by the suppliers of HPMC capsules in several symposia.
A European patent application (EP 0606486) has been submitted claiming their possible use in inhaled formulation because of their lower adherence than hard gelatin capsules. Tests made by applicant have shown that when using conventional dry inhalation system with conventional piercing system, after inhalation, the amount of drug still present in the HPMC capsule (about 20% of the drug initially present in the capsule) was substantially equal to the amount of drug still present in the gelatine capsule (about 18% of the drug initially present in the capsule). Tests made by Applicant have also shown that when using a conventional inhaler system, the lung deposition was better when using a gelatin capsule, instead of a HPMC capsule

In the present invention, it has been discovered that the use of HPMC capsules is not a sufficient condition to obtain better performances than hard gelatin capsules with DPI formulations. On the contrary, as described later, the performances in term of lung deposition of the drug may lower with HPMC capsules than with hard gelatin capsules if the device used for administering the drug is not adapted at the same time.

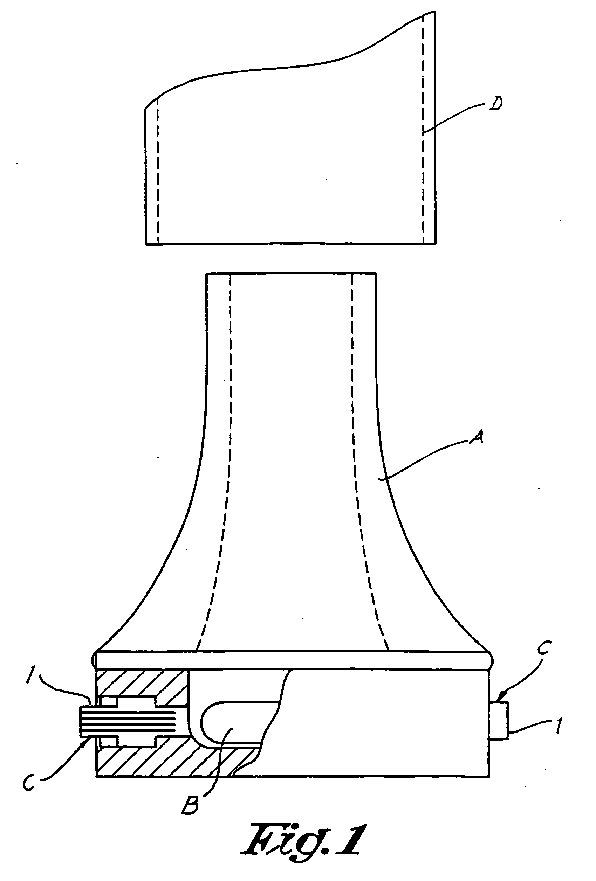

The invention relates principally to the improved lung deposition as quantified by the Fine Particle Dose (FPD) with a combination of (A) a micronized active ingredient mixed or not with excipients, and contained in HPMC capsules and (B) a single dose dry powder inhaler device equipped with at least one piercing pin having a diameter of at least 0.8 mm.
Briefly, the new adapted single dose DPI device is derived from a previous patented device (US 3,991,761). The DPI device described in US patent 3,991,761 contains (A) a mouthpiece, (B) a capsules chamber where the capsule is inserted before inhalation and (C) a perforating system constituted of four pins (or needles) at each end of the capsule chamber in order to perforate 8 holes in the capsules (4 holes at each end of the capsule). Those pins are operated or moved by pressing buttons 1 located at the exterior part of the capsules chamber. The diameter of the 8 piercing pins of this device is of 0.6 ± 0.1 mm. A Figure representing this device is given schematically in figure 1 with the cap (D) removed or in open position. In the present specification, this device will be called the "four pins device". This device is not a device suitable for the combination of the invention.

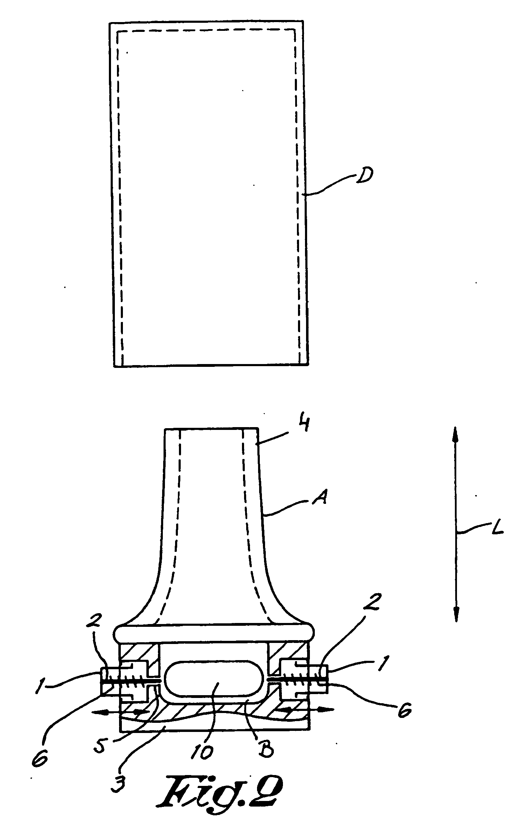

The single dose device of the present invention is derived from the four pin device. The four pins of diameter of 0.6 ± 0.1 mm at both ends of the capsule chamber were replaced by a single pin at each end of the capsule chamber (2 pins in total) but with a larger diameter 1.2 ± 0.1 mm Those bigger pins are bevel-edged in order to allow an easier penetration of the pins in the capsules while the pins of the "four pins device" are "nail-shaped". The single dose device of the present invention with two opposite pins (one for piercing a first end of the capsule and another for piercing the opposite end of the capsule) will be called the " single pin device" in the present specification. The single pin device is schematically represented in figure 2, said device corresponding to the device of figure 1, except that each button 1 is provided with a single larger piercing pin 2, instead of four smaller piercing pins.
It should also be noted that the diameter of the hole made by the piercing pin in hard gelatin or HPMC material is always smaller than the diameter of the pin itself because of the elastic properties of the capsules material, which is responsible for some retraction of the holes after piercing. For instance with the single pin device equipped with pins of diameter of about 1.2 mm, the diameter of the hole observed in the HPMC capsule after piercing is of approximately 1 mm.
Another difference between both kinds of devices is the length L of the mouthpiece A which is shorter on the "single pin device" (33 ± 2 mm) than in the "four pins device" (48 ± 2 mm). It should be noted that the present invention does not relate to a specific kind of device as such, but to the advantageous combination of a specific device and a specific powder container resulting in an optimal inhalation system.
A short description of the "single pin device" will now be given.

The device of figure 2 comprises a mouth piece 4 with a substantially oval end attached to a base element 3, a cap D being provided for covering the mouth piece when the device is not used for inhalation.

The base element 3 comprises a central chamber B adapted for receiving a HPLC capsule, said chamber having a form and a volume greater than the capsule, so as to enable a movement of the capsule during the inhalation.
The base element is provided with two opposite recesses 4, each recess communicating with the chamber B by a channel 5. Each recess is associated to a button 1 bearing a pin 2, the free end is partly engaged in the channel 5 when the button is not operated. The buttons are each operated against the action of a return mechanism, such as a spring 6. When the buttons are pressed the one towards the other, the free end of each pin 2 enters the chamber B so as to contact a capsule placed in said chamber and so as to pierce said capsule. After piercing, the return mechanism enables the movement of the free end of the pin 2 outside of the pierced capsule.
The base element is also provided with means for entering inhalation air in the device. Said air enters the base through the openings 7 and is guided in and/or above the chamber B so as to put into movement (such as rotation) the capsule and so as to aspire the particle and to move them towards the mouth piece A.

Both kind of devices ("four pins device" and "single pin device") work in the same way.
Basically, the DPI device works as follows : the patient opens the device, inserts a capsule in the capsule chamber, closes the device, pushes the buttons in order to pierce the capsule and finally the patient puts the mouthpiece of the device in its mouth and inhales deeply. When the patient inhales, the capsules begins to rotate on itself, so allowing the powder to go outside the capsule via the eight holes pierced by the device of figure 1 or two holes in the case of the single pin device of figure 2 and, via the mouthpiece, to reach the patient's respiratory tract.

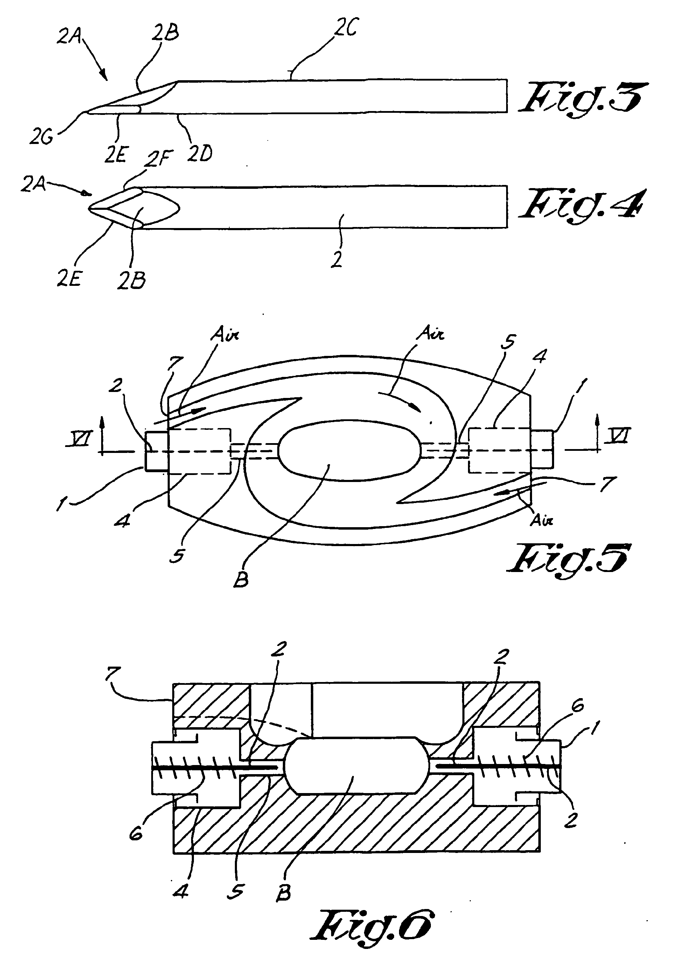

Figures 3 and 4 are enlarged longitudinal views of a pin 2 used in the device of figure 1. The views are a vertical longitudinal view and a horizontal longitudinal view.

The pin 2 is provided with a shaped cutting free end 2A. Said end 2A is provided with an inclined or beveled surface 2B extending between two opposite longitudinal portions (upper and lower portions) 2C,2D of the pin, the angle formed by said surface with respect to the portion 2C,2D being for example comprised between 15 and 75°. The inclined surface is provided with cutting edges 2E,2F connected in the prolongation of the portion 2D by a sharp pointed edge 2G.

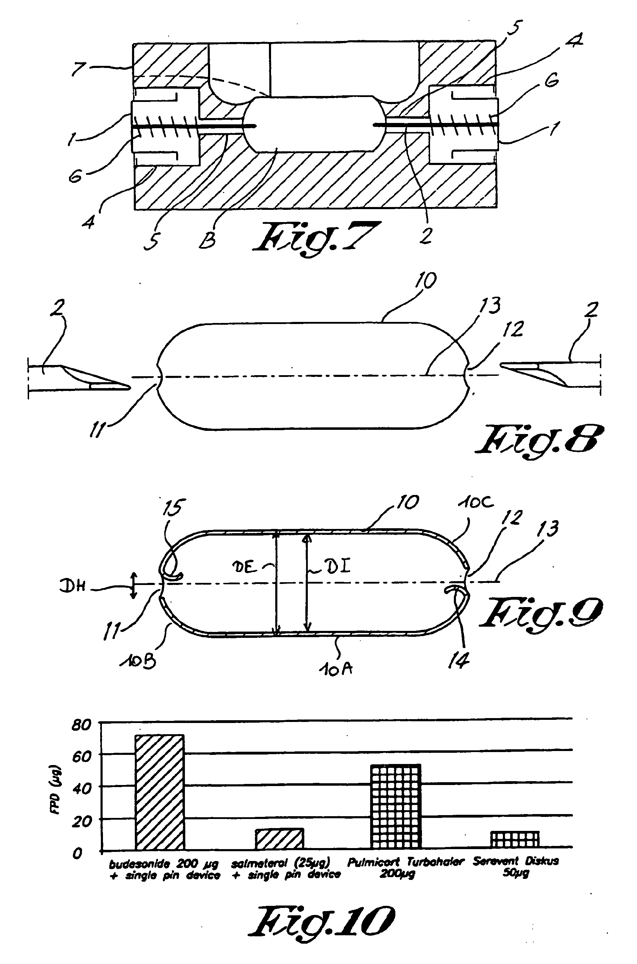

Figure 8 shows on an enlarged scale the piercing or perforation of an envelope HPMC 10. After piercing, the envelope 10 is provided with two opposite openings 11,12 extending substantially along a longitudinal axis 13 of the envelope. The piercing has been made by using two pin as disclosed in figures 3 and 4.

When a pin 2 is entering the envelope 10, the cutting edges 2E,2F form a cutting, while the beveled or inclined surface 2B pushes the cut area of the capsule into the inner space of the capsule, so as to form a small inner guiding surface 14, 15 associated to each opening 11,12.

The diameter DH of the substantially circular opening 11 and 12 (diameter of the open section perpendicular to the axis 13, diameter measured after removal of the pin) corresponds to about 20 to 25% of the inner diameter DI of the longitudinal portion (cylindrical) 10A of the capsule located between the curved ends 10B, 10C, or to about 19 to 24% of the outer diameter DE of the central portion 10A of the capsule 10. Examples of possible capsules are with a size 1, 2 and 3 (for example Vcaps TM), size 2 and 3 being more preferred. The total length of the capsule is for example comprised between 15mm and 20mm, while the outer diameter of the central portion 10A is comprised between 5.5mm and 7mm, the thickness of the wall being about 100µm. The inner volume of the capsule is advantageously lower than 0.5ml, such as 0.3ml. A volume of about 0.3 ml seems to be quite appropriate. The weight of the capsule without active ingredient (i.e. the weight of the capsule as such is advantageously lower than 80mg, preferably lower than 50mg. According to a preferred embodiment, the the total weight of the capsule with the powder to be inhaled is lower than 100mg, most specifically lower than 75mg.

When using pins 2 of figure 3 placed so that the inclined or beveled surfaces are directed towards opposite direction (as shown if figure 8), the inner guiding surface 14 of the hole 12 is directed downwardly, while the inner guiding surface 15 of the hole 11 is directed upwardly, i.e. are directed in opposite directions.

The invention relates thus also to the use of pins as disclosed in figures 3 and 4 for piercing a capsule (HPMC or not), as well as the use of two pins as shown in figure 8 for piercing a capsule (HPMC, gelatin, etc.), and finally also a pierced capsule as shown if figures 8 and 9.

### Example 1 : Formoterol DPI formulation

Formoterol fumarate is a well known long acting bronchodilator used in the treatment of asthma. Formoterol has been formulated in DPI with the formula given herebelow (Table 2) and then the powder was filled into either hard gelatin capsules or HPMC capsules. The FPD of formoterol obtained from each type of capsule is given in Table 3. (The definition of the FPD is given in the European Pharmacopoeia, 3^{rd} edition, chapter 2.9.18. Briefly, the FPD is the dose (expressed in unity of mass) of the drug presenting a diameter below 5.0 µm when a formulation is tested on an Impactor)

The average (average in weight) particle size of the formoterol containing powder was about 3µm (median Gauss range : about 2 to 4 µm, i.e. 50% by weight of the particles have a size comprised between about 2µm and about 4µm).

**Table 2: DPI formulation of formoterol fumarate DPI**

| | **mg/ capsule** |
|---|---|
| micronized formoterol fumarate | 0.012 |
| lactose | 23.988 |
| **TOTAL** | **24.000** |

The in-vitro deposition tests have been realized in the following conditions:
- Impactor: Multistage Liquid Impinger (Eur.Ph. 3^{rd} ed., 2.9.18)
- airflow: 100 l/min
- volume of air: 4 liters
- DPI device: four pins device
- 10 capsules/test
- size 3 capsules: Hard gelatin capsules (Capsugel, Belgium)
   HPMC capsules (Shionogi Qualicaps, Japan)

The tests and the calculations have been performed in accordance with Eur.Ph. 3^{rd} ed., 2.9.18.

**Table 3: FPD obtained with formoterol fumarate DPI formulations with hard gelatin capsules or HPMC capsules + four pins device**

| | **FPD (ug) (mean ± SD)** |
|---|---|
| hard gelatin capsules capsules | 2.63 ± 0.16 |
| HPMC capsules | 2.24 ± 0.12 |

Surprisingly, when a given DPI formulation fumarate of formoterol was filled in respectively hard gelatin capsules or HPMC capsules and administered with the "four pin device", the Fine Particle Dose (FPD) which is representative of the in vitro lung deposition (see definition hereinbelow) measured on a Multistage Liquid Impinger (EP 4^{th} edition, chapter 2.9.18. - apparatus C) was higher for the powder filled into hard gelatin capsules than in HPMC capsules.
Those results are contrary to the teachings of the EP patent 606486 because, as described in Table 1, the HPMC capsules have some potential advantages over hard gelatin capsules (low water content, lower adherence,...) which make them theoretically more performant than the hard gelatin capsules for administering DPI formulations. But the results show the superiority to hard gelatin capsules.

A more detailed look to the holes perforated in the capsules by the device's pins allow to observe that the holes made in hard gelatin capsules were significantly larger than the holes made in HPMC capsules. This observation can explain the higher FPD value obtained with hard gelatin capsules.

On the other hand, it has also been observed that the shape of the holes perforated in HPMC capsules was more regular than the shape of the holes perforated in hard gelatin capsules.
The conclusion of this experiment, was that with the four pin device equipped with pins of a diameter of 0.6 ± 0.1 mm, the holes were smaller but presenting a more regular shape with HPMC capsules than with hard gelatin capsules, resulting in a lower FPD value for HPMC capsules.

The same hard gelatin and HPMC capsules containing the same formoterol fumarate formulation have again been tested on a MLI apparatus (in the same conditions as previously) but this time, they were administered using the "single pin device". Table four summarizes the results of FPD obtained.

**Table 4 : FPD values obtained with formoterol fumarate formulation in HPMC capsules + single pin device or hard gelatin capsule + single pin device**

| | **FPD (ug) (mean ± SD)** |
|---|---|
| hard gelatin capsules capsules | 2.67 ± 0.12 |
| HPMC capsules | 3.25 ± 0.20 |

It should first be noted that the results obtained with the single pin device are different than those obtained with the four pins device,especially for HPMC capsules. Surprisingly enough, this time the FPD obtained from HPMC capsules + single pin device are higher than those obtained with hard gelatin capsules + single pin device. The results obtained with the combination HPMC capsules + single pin device were significantly higher than the results obtained with HPMC capsules + four pin device. The conclusion of this experiment is that the combination described in the present invention i.e. HPMC capsules + single pin DPI devices allows to obtain an higher FPD value and hence the highest in vitro lung deposition. Neither the HPMC capsules alone, nor the single pin device alone was sufficient to provide this high lung deposition. Only the combination of both parameters to form an integrated inhalation system allowed to improve the lung deposition of the drug.

### Example 2 Budesonide

Budesonide is a corticosteroid derivative very widely used in the treatment of asthma. A comparison between a DPI formulation of budesonide (see table 5) filled into HPMC capsules and hard gelatin capsules, and administered respectively with the four pins devices and the single pin device, has been made.

The in-vitro deposition tests have been realized as follows:
- Impactor: Multistage Liquid Impinger
- Airflow: 100 L/min
- Volume of air: 4 liters
- DPI device: four pins device or single pin device
- 3 capsules/test

The tests and calculations have been performed in accordance with Eur. Ph., 3^{rd} ed., 2.9.18.

The formulations of budesonide tested are described in Table 4. The average (average in weight) particle size of the micronized budesonide powder was about 3µm (median Gauss range : about 2 to 4 µm, i.e. 50% by weight of the particles have a size comprised between about 2µm and about 4µm).

**Table 5: formulation of budesonide DPI**

| | **mg/capsule** |
|---|---|
| micronized budesonide | 0.200 |
| lactose | 23.800 |
| **TOTAL:** | **24.00** |

Table 6 gives the result of FPD, MMAD and GSD obtained with budesonide DPI formulations with :
hard gelatin capsules capsules + single pin device
HPMC capsules + single pin device
hard gelatin capsules capsules + four pins device
HPMC capsules + four pins device

The MMAD is the Mass Media Aerodynamic diameter. MMAD is the diameter corresponding to 50% of the cumulative deposition obtained on the Multistage Liquid Impinger. The GSD is the geometric standard deviation of the drug. All the calculations of those parameters have been realized in accordance with Eur. Ph., 3^{rd}, 2.9.18.

**Table 6: comparative in vitro deposition of budesonide DPI formulations in either hard gelatin capsules capsules + single pin device or HPMC capsules + single pin device**

| | **FPD (ug) mean ± SD** | **MMAD mean ± SD** | **GSD mean ± SD** |
|---|---|---|---|
| | **single pin device** | | |
| hard gelatin capsules | 66.3 ± 6.4 | 2.71 ± 0.34 | 1.93 ± 0.08 |
| HPMC | 80.4 ± 6.1 | 2.02 ± 0.24 | 1.82 ± 0.06 |

| | **four pins device** | | |
|---|---|---|---|
| hard gelatin capsules | 65.3 ± 5.4 | 2.85 ± 0.56 | 1.96 ± 0.10 |
| HPMC | 59.4 ± 7.1 | 2.98 ± 0.16 | 2.02 ± 0.06 |

It is clearly demonstrated that the lung deposition of the drug is optimal (high FPD and lower MMAD) when the DPI formulations are filled into HPMC capsules and administered with the single pin device. Another consequence of the single pin device is the lower inter-test variability (lower SD), probably due to the fact that the hole pierced in the capsule with the single pin device is bigger, so allowing a more regular output of the powder from one capsule to the other.

### Example 3 : salmeterol

A DPI formulation containing 50 µg of salmeterol base (under the form of salmeterol xinafoate and 24.950 mg of lactose, has been filed into HPMC capsules. A MLI test has been performed on those capsules administered with the single pin device and the results were compared to the results obtained with a marketed salmeterol DPI formulation of salmeterol (Serevent^{®}, Diskus^{®}, Glaxo Smithkline). Each device was used at the airflow recommended by the european Pharmacopoeia 4^{th} edition i.e 100 Umin for the single pin device and 80 Umin for the Serevent^{®} Diskus^{®}. The results obtained with

**Table 7 : comparative in vitro deposition of salmeterol DPI formulations + single pin device versus Serevent^{®} Diskus^{®} (MLI, n=3)**

| | **FPD (ug) mean ± SD** | **MMAD mean ± SD** | **GSD mean ± SD** |
|---|---|---|---|
| Salmeterol DPI + single pin device | 17.87 ± 0.68 | 2.69 ± 0.05 | 1.37 ± 0.02 |
| Serevent Diskus | 7.89 ± 0.53 | 2.98 ± 0.04 | 1.38 ± 0.01 |

The results clealry demonstrate that the FPD is much higher (more than twice as high) for the salmeterol DPI formulation + single pin than for the marketed formulation and device of salmeterol xinafoate.

### Example 4 : DPI combination of salmeterol / budesonide

A new combination containing 2 active ingredients i.e. budesonide and salmeterol xinafoate in the same DPI capsule has also been tested and compared to the existing DPI formulations of each active ingredient i.e. Pulmicort^{®} Turbuhaler 200 ug (Astra Zeneca) for budesonide and Serevent^{®} Diskus 50 µg for salmeterol xinafoate. Each HPMC capsule of the formulation tested in the object of the present invention contains 200 µg of micronized budesonide, 36.3 µg of micronized salmeterol xinafoate (= 25 µg of salmeterol base) and 23.775 mg of lactose. The HPMC capsule filled with this powder blend was tested on the MLI apparatus using the single dose device of the present invention.
The in-vitro deposition tests have been realized in the following conditions:
- Impactor: Multistage Liquid Impinger (Eur.Ph. 3^{rd} ed., 2.9.18)
- airflow: 100 l/min
- volume of air: 4 liters
- DPI device: four pins device
- 10 capsules/test
- size 3 capsules: HPMC capsules (Shionogi Qualicaps, Japan)

The tests and the calculations have been performed in accordance with Eur.Ph. 3^{rd} ed., 2.9.18.

Figure 10 shows a comparison of various FPD, namely the FPD of budesonide/ salmeterol 200 / 25 µg DPI combinations + single pin device versus Serevent^{®} Diskus^{®} 50 µg and Pulmicort^{®} Turbuhaler 200 µg (MLI).

As seen in the figure 10, the FPD obtained for budesonide and salmeterol are significantly higher for the combination filled into HPMC capsules and administered with the single pin DPI device of the invention than for the respective marketed form of budesonide and salmeterol.

The invention also relates to the improvement of the chemico-physical stability of the DPI powder contained in the HPMC capsules in comparison with the stability obtained with DPI contained in hard gelatin capsules.

This improvement in the chemico-physical stability of DPI powder in HPMC capsules is partially explained by the relatively low content in water of HPMC capsules (3-7%) in comparison with hard gelatin capsules (12-16%). That means that HPMC capsules may be theoretically advantageous for all active ingredients sensitive to moisture. In particular, proteins and active peptides may be advantageously formulated as DPIs using HPMC capsules.

It should be noted that, even if the active ingredient is not chemically sensitive to moisture, it may advantageously be formulated as a DPI in HPMC capsules since the humidity contained in the capsule may also be responsible for agglomeration and/or hygroscopic particles growth, causing a diminution of the FPD and consequently a diminution of the dose available in the patient's lung.

Furthermore, HPMC capsules may be dried until they contain less than 0.5% of water without presenting any apparition of brittleness while hard gelatin capsules capsule became to break themselves when their content in water is below 10%.

The system of the invention can be used for treatment a disease or for preventing troubles, in which a capsule of the invention is used for the administration of one or more active agents, for example for treating respiratory diseases and/or for preventing respiratory troubles.

In use, one or more active ingredients are administered or deposited in the lung(s), and/or in the systemic circulation.

## Claims

1. A dry powder inhalation system comprising:
(A) at least one micronized active ingredient, optionally in association with excipients, contained in a container, advantageously capsule, said container having an outer surface extending between two end portions intended to be pierced or perfored, and
(B) an dry powder inhaler device comprising a mouthpiece (4), a chamber (B) in which the container is inserted, a perforating system with piercing systems able to pierce or perfore said container at said two end portions, and a return mechanism (6) so as to remove the piercing systems outside of the pierced container, whereby the chamber has a form and a volume greater than the container so as to enable, after removal of the piercing systems outside the container, a rotation of the container during the inhalation,
**characterized in that** the container is an hydroxypropylmethylcellulose container, and **in that** the perforating system comprises at least two advantageously substantially identical piercing systems, said piercing systems having an equivalent diameter of not less than 0.8 millimeter (mm), preferably not less than 1 mm, whereby the piercing systems are adapted so that the equivalent diameter of the hole or holes pierced by each piercing system after removal of the piercing system from the hole or holes is from 10 to 31 %, and preferably from 15 % to 26 % of the equivalent diameter of the cross section of the portion of the outer surface of the containe to be pierced located between the two pierced end portions, said cross section being located in a plane perpendicular to an axis extending between the end portions, advantageously a symmetrical axis extending between the end portions.

2. The dry powder inhalation system of claim 1, wherein said piercing systems are bevel-edged needles or pins

3. The dry powder inhalation system of claim 1, wherein the number of said piercing systems per device is less than 8, preferably less than 5, more preferably less than 3.

4. The dry powder inhalation system of claim 1, wherein the active ingredient is mixed with pharmaceutical acceptable carrier before being filled in said container

5. The dry powder inhalation system of claim 4, wherein said pharmaceutical acceptable carrier is a mono- or disaccharide derivative

6. The dry powder inhalation system of claim 4, wherein said pharmaceutical acceptable carrier is lactose

7. The dry powder inhalation system of claim 1, wherein the mean size of the micronized active(s) ingredient(s) is below 10 µm, preferably below 8 µm and most preferably below 6 µm.

8. The dry powder inhalation system of claim 1, wherein the micronized active ingredient is from the class of mucolytics, bronchodilators, corticosteroids, xanthine derivatives, leukotriene antagonists, proteins or peptides, and mixtures thereof.

9. The dry powder inhalation system of claim 1, wherein the active ingredient is L-lysine N-acetylcysteinate.

10. The dry powder inhalation system of claim 1, wherein the dry powder composition contains two or more active ingredients.

11. The dry powder inhalation system of claim 1, with two piercing systems comprising each a single pin, wherein the inhalation system is composed of at least one buccal piece and one basal piece adapted for containing the container or capsule, said basal piece being equipped with the piercing systems and at least one pressing button for operating the pins of the piercing systems.

12. The dry powder inhalation system of claim 1, in which the equivalent diameter of the hole or holes pierced by each piercing system after removal of the piercing system from the hole or holes is from 9 to 30 %, and preferably from 14 % to 25 % of the equivalent inner diameter of the inner cross section of the portion of the outer surface of the container to be pierced located between the two pierced end portions, said cross section being located in a plane perpendicular to an axis extending between the end portions, advantageously a symmetrical axis extending between the end portions

13. The dry powder inhalation system of claim 1, in which the container has an elongated outer surface with a longitudinal axis of symmetry, said outer surface extending between two curved end portions intended to be pierced or perfored, and in which the piercing systems are adapted so that the equivalent diameter of the holes pierced by each piercing system after removal of the piercing system from the hole is from 10 to 31 %, and preferably from 15 % to 26 % of the average equivalent diameter of the cross section of the portion of the outer surface of the container perpendicular to the longitudinal axis of symmetry.

14. The dry powder inhalation system of claim 13, in which the equivalent diameter of the hole pierced by each piercing system after removal of the piercing system from the hole is from 9 to 30 %, and preferably from 14 % to 25 % of the equivalent inner diameter of the inner cross section of the portion of the outer surface of the container to be pierced located between the two pierced end portions, said cross section being located in a plane perpendicular to an axis extending between the end portions, advantageously a symmetrical axis extending between the end portions.

## Patentansprüche

1. Trockenpulver-Inhalationssystem, umfassend
(A) zumindest einen mikronisierten aktiven Inhaltsstoff, optional in Verbindung mit Trägerstoffen, der in einem Behälter, vorteilhaft einer Kapsel, enthalten ist, wobei der Behälter eine Außenfläche aufweist, die sich zwischen zwei Endabschnitten erstreckt, welche zur Durchbohrung oder Perforation bestimmt sind, und
(B) eine Trockenpulver-Inhalatorvorrichtung, umfassend ein Mundstück (4), eine Kammer (B), in die der Behälter eingesetzt wird, ein Perforationssystem mit Durchbohrungssystemen, die fähig sind, den Behälter an den beiden Endabschnitten zu durchbohren oder zu perforieren, und einen Rückholmechanismus (6), um die Durchbohrungssysteme aus dem durchbohrten Behälter zu entfernen, wobei die Kammer eine Form und ein Volumen aufweist, die größer als jene des Behälters sind, um nach dem Entfernen der Durchbohrungssysteme aus dem Behälter eine Drehung des Behälters während der Inhalation zu ermöglichen,
**dadurch gekennzeichnet, dass** der Behälter ein Hydroxypropylmethylcellulosebehälter ist, und dass das Durchbohrungssystem zumindest zwei vorteilhaft im Wesentlichen identische Durchbohrungssysteme umfasst, wobei die Durchbohrungssysteme einen Äquivalenzdurchmesser von nicht weniger als 0,8 Millimeter (mm), und vorzugsweise nicht weniger als 1 mm aufweisen, wobei die Durchbohrungssysteme so angepasst sind, dass der Äquivalenzdurchmesser des Lochs oder der Löcher, die durch jedes Durchbohrungssystem gebohrt werden, nach dem Entfernen des Durchbohrungssystems aus dem Loch oder den Löchern von 10 bis 31 %, und vorzugsweise von 15 % bis 26 % des Äquivalenzdurchmessers des Querschnitts des Abschnitts der Außenfläche des zu durchbohrenden Behälters, der sich zwischen den beiden durchbohrten Endabschnitten befindet, beträgt, wobei sich der Querschnitt in einer Ebene rechtwinkelig zu einer Achse, die sich zwischen den Endabschnitten erstreckt, vorzugsweise einer Symmetrieachse, die sich zwischen den Endabschnitten erstreckt, befindet.

2. Trockenpulver-Inhalationssystem nach Anspruch 1, wobei die Durchbohrungssysteme schrägkantige Nadeln oder Stifte sind.

3. Trockenpulver-Inhalationssystem nach Anspruch 1, wobei die Anzahl der Durchbohrungssysteme pro Vorrichtung geringer als 8, vorzugsweise geringer als 5, und insbesondere geringer als 3 ist.

4. Trockenpulver-Inhalationssystem nach Anspruch 1, wobei der aktive Inhaltsstoff mit einem pharmazeutisch annehmbaren Träger gemischt wird, bevor er in den Behälter gefüllt wird.

5. Trockenpulver-Inhalationssystem nach Anspruch 4, wobei der pharmazeutisch annehmbare Träger ein Mono- oder Disaccharidderivat ist.

6. Trockenpulver-Inhalationssystem nach Anspruch 4, wobei der pharmazeutisch annehmbare Träger Lactose ist.

7. Trockenpulver-Inhalationssystem nach Anspruch 1, wobei die mittlere Größe des mikronisierten aktiven Inhaltsstoffs bzw. der mikronisierten aktiven Inhaltsstoffe unter 10 µm, vorzugsweise unter 8 µm, und insbesondere unter 6 µm liegt.

8. Trockenpulver-Inhalationssystem nach Anspruch 1, wobei der aktive Inhaltsstoff aus der Klasse von Mukolytika, Bronchodilatatoren, Corticosteroiden, Xanthinderivaten, Leukotrienantagonisten, Proteinen oder Peptiden, und Gemischen davon stammt.

9. Trockenpulver-Inhalationssystem nach Anspruch 1, wobei der aktive Inhaltsstoff L-Lysin N-Acetylcysteinat ist.

10. Trockenpulver-Inhalationssystem nach Anspruch 1, wobei die Trockenpulverzusammensetzung zwei oder mehr aktive Inhaltsstoffe enthält.

11. Trockenpulver-Inhalationssystem nach Anspruch 1 mit zwei Durchbohrungssystemen, die jeweils einen einzelnen Stift umfassen, wobei das Inhalationssystem aus zumindest einem Mundstück und einem Basisstück, das zur Aufnahme des Behälters oder der Kapsel geeignet ist, besteht, wobei das Basisstück mit den Durchbohrungssystemen und zumindest einem Druckknopf zum Betätigen der Stifte der Durchbohrungssysteme ausgestattet ist.

12. Trockenpulver-Inhalationssystem nach Anspruch 1, wobei der Äquivalenzdurchmesser des Lochs oder der Löcher, die durch jedes Durchbohrungssystem gebohrt werden, nach dem Entfernen des Durchbohrungssystem aus dem Loch oder den Löchern von 9 bis 30 %, und vorzugsweise von 14 % bis 25 % des Äquivalenzinnendurchmessers des inneren Querschnitts des Abschnitts der Außenfläche des zu durchbohrenden Behälters, der sich zwischen den beiden durchbohrten Endabschnitten befindet, beträgt, wobei sich der Querschnitt in einer Ebene rechtwinkelig zu einer Achse, die sich zwischen den Endabschnitten erstreckt, vorzugsweise einer Symmetrieachse, die sich zwischen den Endabschnitten erstreckt, befindet.

13. Trockenpulver-Inhalationssystem nach Anspruch 1, wobei der Behälter eine langgestreckte Außenfläche mit einer Längssymmetrieachse aufweist, wobei sich die Außenfläche zwischen zwei gekrümmten Endabschnitten erstreckt, die zur Durchbohrung oder Perforation bestimmt sind, und wobei die Durchbohrungssysteme so angepasst sind, dass der Äquivalenzdurchmesser der Löcher, die durch jedes Durchbohrungssystem gebohrt werden, nach dem Entnehmen des Durchbohrungssystems aus dem Loch von 10 bis 31 %, und vorzugsweise von 15 % bis 26 % des durchschnittlichen Äquivalenzdurchmessers des Querschnitts des Abschnitts der Außenfläche des Behälters rechtwinkelig zur Längssymmetrieachse beträgt.

14. Trockenpulver-Inhalationssystem nach Anspruch 13, wobei der Äquivalenzdurchmesser des Lochs, das durch jedes Durchbohrungssystem gebohrt wird, nach dem Entfernen des Durchbohrungssystems aus dem Loch von 9 bis 30 %, und vorzugsweise von 14 % bis 25 % des Äquivalenzinnendurchmessers des inneren Querschnitts des Abschnitts der Außenfläche des zu durchbohrenden Behälters, der sich zwischen den beiden durchbohrten Endabschnitten befindet, beträgt, wobei sich der Querschnitt in einer Ebene rechtwinkelig zu einer Achse, die sich zwischen den Endabschnitten erstreckt, vorzugsweise einer Symmetrieachse, die sich zwischen den Endabschnitten erstreckt, befindet.

## Revendications

1. Système d'inhalation de poudre sèche comprenant :
(A) au moins un principe actif micronisé éventuellement en association avec des excipients, contenu dans un réceptacle, avantageusement une gélule, ledit réceptacle ayant une surface externe s'étendant entre deux parties d'extrémité destinées à être percées ou perforées, et
(B) un dispositif inhalateur de poudre sèche comprenant un embout (4), une chambre (B) dans laquelle le réceptacle est inséré, un système de perforation avec des systèmes de perçage capables de percer ou de perforer ledit réceptacle auxdites deux parties d'extrémité et un mécanisme de rappel (6) de manière à retirer les systèmes de perçage hors du réceptacle percé, la chambre ayant une forme et un volume plus grands que ceux du réceptacle de manière à permettre, après le retrait des systèmes de perçage hors du réceptacle, une rotation du réceptacle pendant l'inhalation,
**caractérisé en ce que** le réceptacle est un réceptacle d'hydroxypropylméthylcellulose et **en ce que** le système de perforation comprend au moins, de manière avantageuse, deux systèmes de perçage sensiblement identiques, lesdits systèmes de perçage ayant un diamètre équivalent non inférieur à 0,8 millimètre (mm), de préférence non inférieur à 1 mm, les systèmes de perçage étant adaptés pour que le diamètre équivalent du ou des trous percés par chaque système de perçage après retrait du système de perçage du ou des trous se situe dans une plage de 10 à 31 %, de préférence de 15 à 26 % du diamètre équivalent de la coupe transversale de la partie de la surface externe du réceptacle à percer située entre les deux parties d'extrémité percées, ladite coupe transversale étant située dans un plan perpendiculaire à un axe s'étendant entre les parties d'extrémité, de manière avantageuse, un axe symétrique s'étendant entre les parties d'extrémité.

2. Système d'inhalation de poudre sèche selon la revendication 1, dans lequel lesdits systèmes de perçage sont des aiguilles ou des pointes à bord biseauté.

3. Système d'inhalation de poudre sèche selon la revendication 1, dans lequel le nombre desdits systèmes de perçage par dispositif est inférieur à 8, de préférence inférieur à 5, mieux encore inférieur à 3.

4. Système d'inhalation de poudre sèche selon la revendication 1, dans lequel le principe actif est mélangé à un véhicule pharmaceutique acceptable avant d'être introduit dans ledit réceptacle.

5. Système d'inhalation de poudre sèche selon la revendication 4, dans lequel ledit véhicule pharmaceutique acceptable est un dérivé de mono- ou disaccharide.

6. Système d'inhalation de poudre sèche selon la revendication 4, dans lequel ledit véhicule pharmaceutique acceptable est le lactose.

7. Système d'inhalation de poudre sèche selon la revendication 1, dans lequel la taille moyenne du ou des principes actifs micronisés est en dessous de 10 µm, de préférence en dessous de 8 µm, bien mieux encore en dessous de 6 µm.

8. Système d'inhalation de poudre sèche selon la revendication 1, dans lequel le principe actif micronisé appartient à la classe des mucolytiques, des bronchodilatateurs, des corticostéroïdes, des dérivés de xanthine, des antagonistes de leucotriènes, des protéines ou des peptides ou de leurs mélanges.

9. Système d'inhalation de poudre sèche selon la revendication 1, dans lequel le principe actif est le N-acétylcystéinate de L-lysine.

10. Système d'inhalation de poudre sèche selon la revendication 1, dans lequel la composition de poudre sèche contient deux principes actifs ou plus.

11. Système d'inhalation de poudre sèche selon la revendication 1, avec deux systèmes de perçage comprenant chacun une pointe unique, dans lequel le système d'inhalation est composé d'au moins une pièce buccale et une pièce de base adaptée pour contenir le réceptacle ou la gélule, ladite pièce de base étant équipée des systèmes de perçage et d'au moins un bouton pression pour l'actionnement des pointes des systèmes de perçage.

12. Système d'inhalation de poudre sèche selon la revendication 1, dans lequel le diamètre équivalent du ou des trous percés par chaque système de perçage après retrait du système de perçage du ou des trous se situe dans une plage de 9 à 30 %, de préférence de 14 à 25 %, du diamètre interne équivalent de la coupe transversale interne de la partie de la surface externe du réceptacle à percer placée entre les deux parties d'extrémité percées, ladite coupe transversale étant placée dans un plan perpendiculaire à un axe s'étendant entre les parties d'extrémité, de manière avantageuse un axe symétrique s'étendant entre les parties d'extrémité.

13. Système d'inhalation de poudre sèche selon la revendication 1, dans lequel le réceptacle a une surface externe allongée avec un axe de symétrie longitudinal, ladite surface externe s'étendant entre deux parties d'extrémité incurvées destinées à être percées ou perforées, et dans lequel les systèmes de perçage sont adaptés pour que le diamètre équivalent des trous percés par chaque système de perçage après retrait du système de perçage du trou se trouve dans une plage de 10 à 31 %, de préférence de 15 à 26 %, du diamètre équivalent moyen de la coupe transversale de la partie de la surface externe du réceptacle perpendiculaire à l'axe de symétrie longitudinal.

14. Système d'inhalation de poudre sèche selon la revendication 13, dans lequel le diamètre équivalent du trou percé par chaque système de perçage après retrait du système de perçage du trou se situe dans une plage de 9 à 30 %, de préférence de 14 à 25 %, du diamètre interne équivalent de la coupe transversale interne de la partie de la surface externe du réceptacle à percer placée entre les deux parties d'extrémité percées, ladite coupe transversale étant placée dans un plan perpendiculaire à un axe s'étendant entre les parties d'extrémité, de manière avantageuse un axe symétrique s'étendant entre les parties d'extrémité.
